# EUROPÄISCHE PATENTSCHRIFT

(11) **EP 2 794 085 B1**
(45) Veröffentlichungstag und Bekanntmachung des Hinweises auf die Patenterteilung: **27.07.2016**
(21) Anmeldenummer: 12791503.1
(22) Anmeldetag: 29.11.2012
(51) Int. Cl.: B01J 13/00

(54) **MIKROKAPSELDISPERSION ENTHALTEND MIKROKAPSELN MIT EINEM HYDROPHILEM KAPSELKERN**
MICROCAPSULE DISPERSION CONTAINING MICROCAPSULES HAVING A HYDROPHILIC CAPSULE CORE
DISPERSION DE MICROCAPSULES CONTENANT DES MICROCAPSULES À NOYAU HYDROPHILE

(30) Priorität: 19.12.2011 EP 11194273
(43) Veröffentlichungstag der Anmeldung: 29.10.2014
(73) Patentinhaber: BASF SE, 67056 Ludwigshafen am Rhein (DE); BASF Schweiz AG, 4057 Basel (CH)
(72) Erfinder: SCHRÖDER - GRIMONPONT , Tina, 76751 Jockgrim (DE)
(86) Internationale Anmeldenummer: PCT/EP2012/073932
(87) Internationale Veröffentlichungsnummer: WO 2013/092158

(56) Entgegenhaltungen:
- DE-A1- 19 749 731
- US-A1- 2010 320 421
- US-A1- 2011 147 961

## Beschreibung

Die vorliegende Erfindung betrifft Mikrokapseldispersionen enthaltend Mikrokapseln umfassend einen hydrophilen Kapselkern und ein Kapselwandpolymer, das erhältlich ist durch Polymerisation einer Monomerzusammensetzung umfassend

| | |
|---|---|
| 25 bis 95 Gew.-% | eines oder mehrerer C₁-C₂₄-Alkyl- und/oder Glycidylester der Acryl- und/oder Methacrylsäure, |
| 5 bis 75 Gew.-% | eines oder mehrerer hydrophiler Monomere ausgewählt unter Acrylsäure- und/oder Methacrylsäureestern, die Hydroxy und/oder Carboxygruppen tragen, und Allylgluconamid |
| 0 bis 40 Gew.-% | einer oder mehrerer Verbindungen mit zwei oder mehr ethylenisch ungesättigten Resten |

wobei die Mikrokapseln in einem hydrophoben Verdünnungsmittel dispergiert sind sowie ein Herstellungs Verfahren und die Verwendung zur verzögerten Freisetzung von Wirkstoffen für Bau-, Kosmetik- oder Pflanzenschutzanwendungen.

Mikrokapseln mit einem hydrophoben Kapselkern kennt man für zahlreiche Anwendungen. Die EP 457 154 lehrt Mikrokapseln mit einem Farbbildner enthaltenden Kernöl und Wänden die durch Polymerisation von Methacrylaten in einer Öl-in-Wasser-Emulsion erhalten werden. In der EP 1029018 werden Mikrokapseln mit Kapselwandpolymeren auf Basis von (Meth)Acrylaten und einem Kapselkern aus lipophilen Wachsen als Latentwärmespeichermaterialien beschrieben. US 2011/147961 A1 offenbart ein Verfahren zur Herstellung einer Mikrokapseldispersion, dadurch gekenntzeichnet, dass man eine hydrophile Kernflüssigkeit-in-Öl-Emulsion enthaltend ein Öl als kontinuierliche Phase sowie eine hydrophile Kernflüssigkeit und eine Monomerzusammensetzung herstellt und anschließend die Monomere radikalisch polymerisiert, wobei die Monomerzusammensetzung (100-20) bis (100-0.1), d.h. 80 bis 99.9 Gew.-% Allylmethacrylat und 0.1 bis 20 Gew.-% Acrylsäure- oder Methacrylsäureestern die Carboxygruppen tragen umfasst. Die hydrophile Kernflüssigkeit ist Wasser.

Weiterhin lehrt die WO 2011/064312 Mikrokapseln mit Pflanzenschutzwirkstoffen gelöst in einem hydrophoben Öl als Kapselkern und ebenfalls einer Kapselwand auf (Meth)Acrylatbasis.

Im Unterschied zu den ÖI-in-Wasser-Emulsionen, bei denen das Öl die disperse, also die diskontinuierliche Phase und das Wasser die kontinuierliche Phase ist, kennt man auch Verkapselungsverfahren, bei denen die beiden Phasen vertauscht sind. Diese Verfahren werden auch als inverse Mikroverkapselung bezeichnet.

Die DE 10120480 beschreibt eine solche inverse Verkapselung. Sie lehrt Mikrokapseln mit einem wasserlösliche Substanzen enthaltenden Kapselkern und einer Kapselwand aus Melamin/Formaldehyd-Harzen. Weiterhin lehrt die of WO 03/015910 Mikrokapseln mit einem wasserlösliche Substanzen enthaltenden Kapselkern und einer Kapselwand aus Polyharnstoffen.

Die EP-A-0 148 169 beschreibt Mikrokapseln mit einem wasserlöslichen Kern und einer Polyurethanwand, die in einem Pflanzenöl hergestellt werden. Als Kapselkernmaterial werden neben Herbiziden unter anderem wasserlösliche Farbstoffe genannt.

So besteht jedoch nach wie vor ein Bedarf an Mikrokapseln mit einem Wasser enthaltenden Kapselkern, die man beispielsweise als Porenbildner in Baumaterialien einsetzen kann. Auch ist es wünschenswert auf diese Weise Säure zu schützen, deren Freisetzung als Beschleuniger für beispielsweise Pressspanplatten man steuern kann. Auch verzögerte Freisetzung von wasserlöslichen Wirkstoffen für Pflanzenschutz- oder Kosmetikanwendungen ist von Interesse.

Aufgabe der vorliegenden Erfindung war es wässrige Lösungen oder auch Wasser selber zu verkapseln.

Demgemäß wurden der oben Beschriebenen Mikrokapseln Dispersionen in einem hydrophoben Verdünnungsmittel sowie ein Herstellungs Verfahren gefunden.

Die Mikrokapseln umfassen einen Kapselkern und eine Kapselwand. Der Kapselkern besteht überwiegend, zu mehr als 95 Gew.-%, aus Wasser oder wässrigen Lösungen. Die mittlere Teilchengröße der Kapseln (Z-Mittel mittels Lichtstreuung) beträgt 0,5 bis 50 µm. Gemäß einer bevorzugten Ausführungsform beträgt die mittlere Teilchengröße der Kapseln 0,5 bis 15 µm, bevorzugt 0,5 bis 10 µm. Dabei haben bevorzugt 90% der Teilchen eine Teilchengröße von weniger als der doppelten mittleren Teilchengröße.

Das Gewichtsverhältnis von Kapselkern zu Kapselwand beträgt im allgemeinen von 50 : 50 bis 95 : 5. Bevorzugt wird ein Kern/Wand-Verhältnis von 70 : 30 bis 93 : 7.

Unter einem hydrophilen Kapselkern (Kapselkernmaterial) sind Wasser sowie wässrigen Lösungen von wasserlöslichen Verbindungen zu verstehen, deren Gehalt mindestens 10 Gew.-% einer wasserlöslichen Verbindung beträgt. Bevorzugt sind die wässrigen Lösungen mindestens 20 gew.-%ig.

Bei den wasserlöslichen Verbindungen handelt es sich beispielsweise um organische Säuren oder deren Salze, anorganische Säuren, anorganische Basen, Salze anorganischer Säuren wie Natriumchlorid oder Natriumnitrat, wasserlösliche Farbstoffe, Agrochemikalien wie Dicamba^{®}, Geschmacksstoffe, pharmazeutische Wirkstoffe, Dünger oder kosmetische Wirkstoffe. Bevorzugt werden als hydrophiles Kapselkernmaterial Wasser sowie wässrigen Lösungen von organischen Säuren wie Essigsäure, Ameisensäure, Propionsäure und Methansulfonsäure, und/oder deren Salze, anorganische Säuren wie Phosphorsäure und Salzsäure, und Salze anorganischer Säuren sowie Natriumsilikat.

Abhängig von der Dicke der Kapselwand, die durch die gewählten Verfahrensbedingungen sowie Menge der Einsatzstoffe beeinflusst wird, sind die Kapseln undurchlässig oder schwer durchlässig für das hydrophile Kapselkernmaterial. Mit schwer durchlässigen Kapseln lässt sich eine kontrollierte Abgabe des hydrophilen Kapselkernmaterials erzielen. Das den Kapselkern bildende Wasser wird in der Regel aus isolierten Mikrokapseln, also vom hydrophoben Verdünnungsmittel befreiten Mikrokapseln im Laufe der Zeit verdunsten.

Sofern im Rahmen dieser Anmeldung von -(meth)acrylaten die Rede ist, sind sowohl die entsprechenden -acrylate, also die Derivate der Acrylsäure, wie auch die - methacrylate, die Derivate der Methacrylsäure zu verstehen.

Die Polymere der Kapselwand enthalten im Allgemeinen mindestens 25 Gew.-%, in bevorzugter Form mindestens 30 Gew.-% und in besonders bevorzugter Form mindestens 40 Gew.-% sowie im Allgemeinen höchstens 95 Gew.-%, vorzugsweise höchstens 90 Gew.-% und in besonders bevorzugter Form höchstens 80 Gew.-% C₁-C₂₄-Alkyl-und/oder Glycidylester der Acryl- und/oder Methacrylsäure (Monomere I) einpolymerisiert, bezogen auf das Gesamtgewicht der Monomere.

Erfindungsgemäß enthalten die Polymere der Kapselwand im Allgemeinen mindestens 5 Gew.-%, bevorzugt mindestens 10 Gew.-%, vorzugsweise mindestens 15 Gew.-% sowie im Allgemeinen höchstens 75 Gew.-%, vorzugsweise höchstens 60 Gew.-% und in besonders bevorzugter Form höchstens 55 Gew.-% eines oder mehrerer hydrophiler Monomere (II) ausgewählt unter Acrylsäureestern, die Hydroxy und/oder Carboxygruppen tragen, Methacrylsäureestern, die Hydroxy und/oder Carboxygruppen tragen, und Allylgluconamid, bezogen auf das Gesamtgewicht der Monomere, einpolymerisiert.

Daneben können die Polymere bevorzugt mindestens 5 Gew.-%, bevorzugt mindestens 10 Gew.-%, vorzugsweise mindestens 15 Gew.-% sowie im Allgemeinen höchstens 40 Gew.-%, vorzugsweise höchstens 35 Gew.-% und in besonders bevorzugter Form höchstens 30 Gew.-% einer oder mehrerer Verbindungen mit zwei oder mehr ethylenisch ungesättigten Resten (Monomere III) einpolymerisiert enthalten, bezogen auf das Gesamtgewicht der Monomere.

Weiterhin können bis zu 5 Gew.-% sonstige Monomere IV, die von den Monomeren I, II und III verschieden sind, in der Kapselwand in einpolymerisierter Form enthalten sein.

Bevorzugt besteht die Monomerzusammensetzung aus den Monomeren I und II sowie gegebenenfalls den Monomeren III sowie gegebenenfalls den Monomeren IV.

Als Monomere I eignen sich C₁-C₂₄-Alkylester der Acryl- und/oder Methacrylsäure sowie die Glycidylester der Acryl- und/oder Methacrylsäure. Bevorzugte Monomere I sind Methyl-, Ethyl-, n-Propyl- und n-Butylacrylat sowie die entsprechenden Methacrylate. Generell werden die Methacrylate bevorzugt. Besonders bevorzugt werden C₁-C₄-Alkylmethacrylate. Gemäß einer weiteren Ausführungsform wird Glycidylmethacrylat bevorzugt.

Gemäß einer besonders bevorzugten Ausführungsform ist Monomer I Methylmethacrylat gegebenfalls in Mischung mit Glycidylmethacrylat und/oder einem oder mehrerer C₂-C₂₄-Alkylestern der Acryl- und/oder Methacrylsäure. Besonders bevorzugt enthält die Monomerzusammensetzung 25-40 Gew.-% Methylmethacrylat.

Monomere II werden ausgewählt unter Acrylsäureester, die Hydroxy und/oder Carboxygruppen tragen, Methacrylsäureester, die Hydroxy und/oder Carboxygruppen tragen, und Allylgluconamid. Bevorzugt handelt es sich um (Meth)-Acrylsäureester, die mindestens einen Rest ausgewählt unter Carbonsäure- und Hydroxyrest tragen. Die bevorzugten (Meth)-Acrylsäureester sind hydrophil, dass heißt sie haben eine Wasserlöslichkeit von >50g/L bei 20°C und Normaldruck.

Als Monomere II werden bevorzugt Hydroxyalkylacrylate und Hydroxyalkylmethacrylate wie 2-Hydroxyethylacrylat und -methacrylat, Hexapropylacrylat und -methacrylat, Hydroxybutylacrylat und Diethylenglycolmonoacrylat eingesetzt.

Weiterhin bevorzugte hydrophile Monomere II sind Acrylamidoalkylpolyhydroxysäureamide, Methacrylamidoalkyl-polyhydroxysäureamide, N-Acryl-Glykosylamine und N-Methacryl-Glykosylamine.

Die Herstellung der Acrylamidoalkyl-polyhydroxysäureamide sowie der Methacrylamidoalkyl-polyhydroxysäureamide ist bekannt und beispielsweise in der WO 2010/118951 beschrieben. Weiterhin ist die Herstellung der N-Acryl-Glykosylamine und N-Methacryl-Glykosylamine bekannt und beispielsweise in der WO 2010/118951 beschrieben.

So erfolgt die Herstellung von N-Acryl-Glykosylaminen bzw. N-Methacryl-Glykosylaminen in zwei Schritten indem man einen Aldehydzucker mit einem primären aliphatischen Amin oder Ammoniak zum korrespondierenden Glykosylamin umsetzt und das entstandene N-Glykosylamin mit dem Acrylsäureanhydrid oder Methacrylsäureanhydrid zum N-Acryl-Glykosylamin bzw. N-Methacryl-Glykosylamin umsetzt. Die beiden Verfahrensschritte werden erfindungsgemäß direkt nacheinander also ohne Zwischenisolierung durchgeführt.

Unter Aldehydzucker sind nachfolgend reduzierende Zucker zu verstehen, die in ihrer offenkettigen Form eine Aldehydgruppe tragen. Die erfindungsgemäß eingesetzten Aldehydzucker sind offenkettige bzw. cyclische Mono- und Oligosaccharide aus natürlichen und synthetischen Quellen mit einem Aldehydrest bzw. dessen Halbacetal. Insbesondere sind die Aldehydzucker ausgewählt unter Mono- und Oligosacchariden in optisch reiner Form bevorzugt. Sie sind auch als Stereoisomerengemisch geeignet.

Monosaccharide sind ausgewählt unter Aldosen, insbesondere Aldo-Pentosen und bevorzugt Aldo-Hexosen. Geeignete Monosaccharide sind beispielsweise Arabinose, Ribose, Xylose, Mannose und Galactose insbesondere Glukose. Da die Monosaccharide in wässriger Lösung umgesetzt werden, liegen sie aufgrund der Mutarotation sowohl in ringförmiger Halbacetal-Form wie auch zu einem gewissen Prozentsatz in offenkettiger Aldehyd-Form vor.

Bevorzugt ist der Aldehydzucker ein Oligosaccharid. Unter Oligosaccharide werden Verbindungen mit 2 bis 20 Wiederholungseinheiten verstanden. Bevorzugte Oligosaccharide sind ausgewählt unter Di-, Tri-, Tetra-, Penta-, und Hexa-, Hepta-, Octa, Nona- und Decasacchariden, bevorzugt Saccharide mit 2 bis 9 Wiederholungseinheiten. Die Verknüpfung innerhalb der Ketten erfolgt 1,4-glycosidisch und gegebenenfalls 1,6-glycosidisch. Die Aldehydzucker haben, auch wenn es sich um oligomere Aldehydzucker handelt, eine reduzierende Gruppe pro Molekül.

Bevorzugt werden als Aldehydzucker (Saccharide) Verbindungen der allgemeinen Formel I eingesetzt 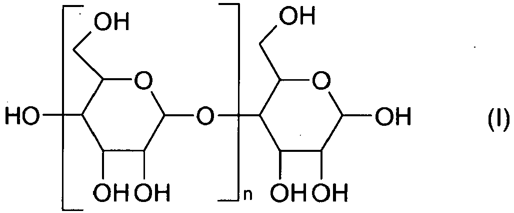 in der n für die Zahl 0, 1, 2, 3, 4, 5, 6, 7 oder 8 steht.

Die Oligosaccharide in denen n für eine ganze Zahl von 1 bis 8 steht, werden besonders bevorzugt. Dabei ist es möglich, Oligosaccharide mit einer definierten Zahl an Wiederholungseinheiten einzusetzen. Beispielhaft seien als Oligosaccharide Lactose, Maltose, Isomaltose, Maltotriose, Maltotetraose und Maltopentaose genannt.

Vorzugsweise werden Mischungen aus Oligosacchariden mit unterschiedlicher Anzahl an Wiederholungseinheiten gewählt. Derartige Mischungen sind durch Hydrolyse eines Polysaccharids bevorzugt von Cellulose oder Stärke erhältlich, wie enzymatische oder sauer katalysierte Hydrolyse von Cellulose oder Stärke. Pflanzliche Stärke besteht aus Amylose und Amylopektin als Hauptbestandteil der Stärke. Amylose besteht aus überwiegend unverzweigten Ketten von Glucosemolekülen, die 1,4-glycosidisch miteinander verknüpft sind. Amylopektin besteht aus verzweigten Ketten, in denen es neben den 1,4-glycosidischen Verknüpfungen zusätzlich 1,6-glycosidische Verknüpfungen gibt, die zu Verzweigungen führen. Erfindungsgemäß eignen sich auch Hydrolyseprodukte von Amylopektin als Ausgangsverbindung für das erfindungsgemäße Verfahren und sind mit von der Definition Oligosaccharide umfasst.

Zur Umsetzung geeignete primäre aliphatische Amine können linear oder verzweigt sein. Primäre aliphatische Amine im Sinne dieser Erfindung sind aliphatische Monoamine, bevorzugt gesättigte Monoamine, mit einer primären Aminogruppe. Der gesättigte aliphatische Rest ist in der Regel ein Alkylrest, mit vorzugsweise 1 bis 8 C-Atomen, der durch O-Atome unterbrochen sein kann und der gegebenenfalls ein oder zwei Carboxylgruppen, Hydroxylgruppen und oder Carboxamidgruppen tragen kann.

Als geeignete primäre aliphatische Amine, die mit Hydroxyl, Carboxyl oder Carboxamid substituiert sind, seien Alkanolamine wie Ethanolamin, und Aminosäuren wie Glycin, Alanin, Phenylalanin, Serin, Asparagin, Glutamin, Asparaginsäure und Glutaminsäure genannt. Geeignete primäre aliphatische Amine, deren Alkylenrest mit Sauerstoff unterbrochen ist, sind bevorzugt 3-Methoxy-propylamin, 2-Ethoxy-ethylamin, und 3-(2-Ethylhexyloxy)propylamin.

Bevorzugt werden als primäre aliphatische Amine C₁-C₈-Alkylamine, insbesondere C₁-C₄-Alkylamine, eingesetzt wie Ethylamin, 1-Amino-propan, 2-Amino-propan, 1- Aminobutan, 2-Amino-butan, insbesondere Methylamin.

Bevorzugt werden die primären aliphatischen Amine ausgewählt unter Methylamin und Ethanolamin. Weiterhin ist die Umsetzung mit Ammoniak bzw. Mischungen von Ammoniak mit primären aliphatischen Aminen bevorzugt.

Die eingesetzten Anhydride sind Methacrylsäureanhydrid und Acrylsäureanhydrid.

Die Herstellung der Acrylamidoalkyl-polyhydroxysäureamide bzw. Methacrylamidoalkyl-polyhydroxysäureamide erfolgt schematisch in zwei Schritten: im ersten Schritt der Reaktion des Polyhydroxysäurelactons mit dem aliphatischen Diamin zum korrespondierenden Aminoalkylaldonamid und im zweiten Schritt der Umsetzung des Aminoalkylaldonamids mit Methacrylsäureanhydrid oder Acrylsäureanhydrid zum erfindungsgemäßen ungesättigten Methacryl- bzw. Acrylamidoalkylpolyhydroxysäureamid. Gegebenenfalls kann eine Zwischenisolierung vorteilhaft sein.

Unter Polyhydroxysäurelacton sind nachfolgend Lactone von lediglich am anomeren Kohlenstoff oxidierten Sacchariden aus natürlicher und synthetischer Quelle zu verstehen. Derartige Polyhydroxysäurelactone können auch als Lactone von Aldonsäuren bezeichnet werden. Die Polyhydroxysäurelactone können einzeln oder in ihren Mischungen eingesetzt werden.

Die Saccharide werden nur am anomeren Zentrum selektiv oxidiert. Verfahren zur selektiven Oxidation sind allgemein bekannt und werden beispielsweise beschrieben in J. Lönnegren, I. J. Goldstein, Methods Enzymology, 242 (1994) 116. So kann man die Oxidation mit Jod im alkalischen Milieu oder mit Kupfer(II)-Salzen durchführen. Geeignete Saccharide sind die obengenannten insbesondere als bevorzugt genannten Saccharide.

Geeignete aliphatische Diamine können linear, cyclisch oder verzweigt sein. Aliphatische Diamine im Sinne dieser Erfindung sind Diamine mit zwei primären oder sekundären Aminogruppen, vorzugsweise mit einer primären und einer weiteren primären oder sekundären Aminogruppe, die durch einen aliphatischen, vorzugsweise gesättigten bivalenten Rest miteinander verbunden sind. Der bivalente Rest ist in der Regel ein Alkylenrest, mit vorzugsweise 2 bis 10 C-Atomen, der durch O-Atome unterbrochen sein kann und der gegebenenfalls ein oder zwei Carboxylgruppen, Hydroxylgruppen und oder Carboxamidgruppen tragen kann. Weiterhin werden unter aliphatischen Diaminen auch cycloaliphatische Diamine verstanden.

Als geeignete aliphatische Diamine, die mit Hydroxyl, Carboxyl oder Carboxamid substituiert sind, seien beispielhaft N-(2-Aminoethyl)-ethanolamin, 2,4-Diaminobuttersäure oder Lysin genannt.

Die geeignete aliphatische Diamine, deren Alkylenrest mit Sauerstoff unterbrochen ist, sind bevorzugt α,ω-Polyetherdiamine, bei denen die beiden Aminogruppen an den Kettenenden des Polyethers sind. Polyetherdiamine sind bevorzugt die Polyether des Ethylenoxids, des Propylenoxids und des Tetrahydrofurans. Die Molekulargewichte der Polyetherdiamine sind im Bereich von 200 - 3000 g/mol, bevorzugt im Bereich von 230 - 2000 g/mol.

Bevorzugt werden aliphatische C₂-C₈-Diamine und cycloaliphatische Diamine eingesetzt, wie 1,2-Diaminoethan, 1,3-Diaminopropan, 1, 5-Diaminopentan, 1,6-Diaminohexan, N-Methyl-1,3-Diaminopropan, N-Methyl-1,2-Diaminoethan, 2,2-Dimethylpropan-1,3-diamin, Diamino-cyclohexan, Isöphorondiamin und 4,4'-Diaminodicyclohexyl-methan.

Verbindungen mit zwei oder mehr ethylenisch ungesättigten Resten (Monomere III) wirken als Vernetzer. Bevorzugt werden Monomere, mit Vinyl-, Allyl-, Acryl- oder/oder Methacrylgruppen verwendet.

Geeignete Monomere I mit zwei ethylenisch ungesättigten Resten sind beispielsweise Divinylbenzol und Divinylcyclohexan und bevorzugt die Diester von Diolen mit Acrylsäure oder Methacrylsäure, ferner die Diallyl- und Divinylether dieser Diole. Beispielhaft seien Ethandioldiacrylat, Ethylenglykoldimethacrylat, 1,3-Butylenglykoldimethacrylat, Diethylenglykoldiacrylat, Dipropylenglykoldiacrylat, Methallylmethacrylamid, Allylacrylat und Allylmethacrylat genannt. Besonders bevorzugt sind Propandiol-, Butandiol-, Pentandiol- und Hexandioldiacrylat und die entsprechenden Methacrylate.

Monomere III mit drei oder mehr, in der Regel 3, 4 oder 5 ethylenisch ungesättigten Resten sind beispielsweise die Polyester von Polyolen mit Acrylsäure und/oder Methacrylsäure, ferner die Polyallyl- und Polyvinylether dieser Polyole. Bevorzugt werden Monomere III mit drei oder mehr ethylenisch ungesättigten Resten wie Trimethylolpröpantriacrylat und -methacrylat, Pentaerythrittriallylether, Pentaerythrittetraallylether, Pentaerythrittriacrylat und Pentaerythrittetraacrylat sowie ihre technischen Mischungen. So liegt Pentaerythrittetraacrylat in der Regel in technischen Mischungen im Gemisch mit Pentaerythrittriacrylat und geringeren Mengen Oligomerisierungsprodukten vor.

Als sonstige Monomere IV kommen monoethylenisch ungesättigte Monomere, die von den Monomeren I und II verschieden sind in Betracht wie Styrol, β-Methylstyrol, Vinylacetat, Vinylpropionat und Vinylpyridin in Betracht.

Besonders bevorzugt sind die wasserlöslichen Monomere IV wie Acrylsäure, Methacrylsäure, Acrylnitril, Methacrylamid, Itaconsäure, Maleinsäure, Maleinsäureanhydrid, N-Vinylpyrrolidon, und Acrylamido-2-methylpropansulfonsäure. Daneben sind insbesondere N-Methylolacrylamid, N-Methylolmethacrylamid, Dimethylaminoethylmethacrylat und Diethylaminoethylmethacrylat zu nennen.

Bevorzugt werden Monomerzusammensetzungen bestehend aus

| | |
|---|---|
| 25 bis 90 Gew.-% | eines oder mehrerer C₁-C₂₄-Alkyl- und/oder Glycidylester der Acryl- und/oder Methacrylsäure, |
| 5 bis 75 Gew.-% | eines oder mehrerer Monomere ausgewählt unter Acrylsäure- und/oder Methacrylsäureestern, die Hydroxy- und/oder Carboxygruppen tragen, und Allylgluconamid |
| 15 bis 40 Gew.-% | eines oder mehrerer Verbindungen mit zwei oder mehr ethylenisch ungesättigten Resten |
| 0 bis 10 Gew.-% | eines oder mehrerer sonstiger Monomere |

zur Bildung des Kapselwandpolymers durch radikalische Polymerisation eingesetzt.

Ebenfalls bevorzugt werden Monomerzusammensetzungen umfassend, bevorzugt bestehend aus

| | |
|---|---|
| 25 bis 95 Gew.-% | eines oder mehrerer C₁-C₂₄-Alkyl- und/oder Glycidylester der Acryl- und/oder Methacrylsäure, |
| 30 bis 75 Gew.-% | eines oder mehrerer Monomere ausgewählt unter Acrylsäure- und/oder Methacrylsäureestern, die Hydroxy- und/oder Carboxygruppen tragen, und Allylgluconamid |
| 0 bis 40 Gew.-% | eines oder mehrerer Verbindungen mit zwei oder mehr ethylenisch ungesättigten Resten |
| 0 bis 5 Gew.-% | eines oder mehrerer sonstiger Monomere |

zur Bildung des Kapselwandpolymers durch radikalische Polymerisation eingesetzt.

Die Mikrokapseln sind erhältlich durch Herstellen einer Wasser-in-Öl-Emulsion enthaltend hydrophobes Verdünnungsmittel als kontinuierliche Phase sowie das hydrophile Kapselkernmaterial und die Monomere und anschließender radikalischer Polymerisation der Monomere zur Bildung des Kapselwandpolymers. Die Monomere können dabei als Mischung eingesetzt werden. Es ist jedoch ebenso möglich sie getrennt, abhängig von ihrer Hydrophilie also Löslichkeit in Wasser, in Mischung mit dem Kapselkernmaterial und in Mischung mit dem hydrophoben Verdünnungsmittel zu dosieren. So werden die Monomere II vorzugsweise in Mischung mit dem hydrophilen Kapselkernmaterial dosiert. Die Monomere I werden vorzugsweise in Mischung mit dem hydrophoben Verdünnungsmittel dosiert.

Die kontinuierliche Phase der Emulsion enthält üblicherweise oberflächenaktive Substanzen, um ein Zusammenfließen der Tröpfchen zu vermeiden. In dieser Emulsion ist das Wasser bzw. die wässrige Lösung die diskontinuierliche spätere disperse Phase und das hydrophobe Verdünnungsmittel die kontinuierliche Phase. Die emulgierten Tröpfchen besitzen dabei eine Größe, die ungefähr der Größe der späteren Mikrokapseln entspricht. Die Wandbildung erfolgt durch die Polymerisation der Monomerzusammensetzung, die durch Radikalstarter gestartet wird.

Als hydrophobes Verdünnungsmittel werden nachfolgend Verdünnungsmittel verstanden, die eine Löslichkeit in Wasser von <1 g/L, bevorzugt < 0,5g/L bei 20°C und Normaldruck aufweisen. Vorzugsweise wird das hydrophobe Verdünnungsmittel ausgewählt unter
- Cyclohexan,
- Glycrinesterölen,
- Kohlenwasserstoff-Öle, wie Paraffinöl, Diisopropylnaphthalin, Purcellinöl, Perhydrosqualen und Lösungen mikrokristalliner Wachse in Kohlenwasserstoff-Ölen,
- tierische oder pflanzliche Öle,
- mineralische Öle, deren Destillationsbeginn unter Atmosphärendruck bei ca. 250 C und deren Destillationsendpunkt bei 410°C liegt, wie z. B. Vaselinöl,
- Ester gesättigter oder ungesättigter Fettsäuren, wie Alkylmyristate, z. B: i-Propyl-, Butyl-oder Cetylmyristat, Hexadecylstearat, Ethyl-oder i-Propylpalmitat und Cetylricinolat.
- Siliconöle, wie Dimethylpolysiloxan, Methylphenylpolysiloxan und das Siliconglycol-Copolymer,
- Fettsäuren und Fettalkohole oder Wachse wie Carnauba-Wachs, Candellilawachs, Bienenwachs, mikrokristallines Wachs, Ozokeritwachs und Ca-, Mg- und Al-Oleate, -Myristate,-Linoleate und-Stearate.

Unter Glycerinesterölen versteht man Ester gesättigter oder ungesättigter Fettsäuren mit Glycerin. Geeignet sind Mono-, Di- und Triglyceride sowie ihre Mischungen. Bevorzugt werden Fettsäuretriglyceride. Als Fettsäuren seien beispielhaft C₆-C₁₂-Fettsäuren wie Hexan-, Octan-, Decan- und Dodecansäure erwähnt. Bevorzugte Glycerinesteröle sind C₆-C₁₂-Fettsäuretriglyceride insbesondere Octan- und Decansäuretriglyceride sowie ihre Mischungen. Eine solche Octanoylglycerid/Decanoylglycerid Mischung ist beispielsweise Miglyol^{®} 812 der Fa. Hüls.

Um eine stabile Emulsion zu erhalten, benötigt man oberflächenaktive Substanzen wie Schutzkolloide und/oder Emulgatoren. In der Regel verwendet man oberflächenaktive Substanzen, die sich mit der hydrophoben Phase mischen.

Bevorzugte Schutzkolloide sind lineare Blockcopolymere mit einer hydrophoben Struktureinheit von einer Länge > 50Å, allein oder in Mischungen mit anderen oberflächenaktiven Substanzen. Die linearen Blockcopolymere werden durch die allgemeine Formel

C_{w}-(-B-A-B_{y}-)-xD_{z}

wiedergegeben, in der w 0 oder 1, x 1 oder mehr, y 0 oder 1 und z 0 oder 1 bedeutet und A eine hydrophile Struktureinheit, mit einer Löslichkeit in Wasser bei 25°C> 1 Gew.-% (> 10g/l) und einem Molekulargewicht von 200 bis 50 000 ist, die kovalent mit den B-Blöcken verbunden ist und B eine hydrophobe Struktureinheit, mit einem Molekulargewicht von 300 bis 60 000 und eine Löslichkeit < 1 Gew. -% in Wasser bei 25°C ist und zu A kovalente Bindungen bilden kann; und in der C und D Endgruppen sind, die unabhängig voneinander A oder B sein können. Die Endgruppen können gleich oder verschieden sein und sind abhängig vom Herstellungsverfahren.

Beispiele für hydrophile Gruppen sind Polyethylenoxide, Poly(1, 3-dioxolan), Copolymere von Polyethylenoxid oder Poly(1, 3-dioxolan), Poly(2-methyl-2-oxazolin), Poly(glycidyltrimethylammoniumchlorid) und Polymethylenoxid.

Beispiele für hydrophobe Gruppen sind Polyester bei denen der hydrophobe Teil eine sterische Barriere ≥ 50 Å, vorzugsweise ≥75 Å insbesondere ≥100 Å ist. Die Polyester sind abgeleitet von Komponenten wie 2-Hydroxybutansäure, 3-Hydroxybutansäure, 4-Hydroxybutansäure, 2-Hydroxycapronsäure, 10-Hydrodecansäure, 12-Hydroxydodecansäure, 16-Hydroxyhexadecansäure, 2-Hydroxyisobutansäure, 2- (4-Hydroxyphenoxy)propionsäure, 4-Hydroxyphenylbrenztraubensäure, 12-Hydroxystearinsäure, 2-Hydroxyvaleriansäure, Polylactonen aus Caprolacton und Butyrolacton, Polylactamen aus Caprolactam, Polyurethanen und Polyisobutylenen. Bevorzugt stabilisiert man die Wasser-in-Öl-Emulsion mit einem 12-Hydroxystearinsäure-Blockcopolymer als linearem Blockcopolymer.

Die linearen Blockcopolymere enthalten sowohl hydrophile wie auch hydrophobe Einheiten. Die Blockpolymere haben ein Molekulargewicht oberhalb 1000 und eine Länge des hydrophoben Teils von ≥50 Å berechnet nach dem Gesetz von Cosines. Diese Größen werden bei ausgestreckter Konfiguration berechnet unter Berücksichtigung der in der Literatur angegebenen Bindungslängen und -winkel. Die Herstellung dieser Einheiten ist allgemein bekannt. Herstellverfahren sind beispielsweise Kondensationsreaktion von Hydroxysäuren, Kondensationen von Polyolen wie Diolen mit Polycarbonsäuren wie Dicarbonsäuren. Geeignet ist auch die Polymerisation von Lactonen und Lactamen sowie die Reaktion von Polyolen mit Polyisocyanaten. Hydrophobe Polymereinheiten werden mit den hydrophilen Einheiten wie allgemein bekannt umgesetzt, beispielsweise durch Kondensationsreaktion und Kupplungsreaktion. Die Herstellung solcher Blockcopolymere wird beispielsweise in der US 4 203 877 beschrieben, auf die ausdrücklich verwiesen wird.

Bevorzugt beträgt der Anteil an linearem Blockcopolymer 20-100 Gew. -% der Gesamtmenge an eingesetzter oberflächenaktiver Substanz.

Geeignete oberflächenaktive Substanzen sind ferner die gewöhnlich für Wasser-in-Öl-Emulsionen verwendeten Emulgatoren beispielsweise
- C₁₂-C₁₈-Sorbitan-Fettsäureester,
- Ester von Hydroxystearinsäure und C₁₂-C₃₀-Fettalkoholen,
- Mono- und Diester von C₁₂-C₁₈-Fettsäuren und Glycerin oder Polyglycerin,
- Kondensate von Ethylenoxid und Propylenglycolen,
- oxypropylenierte/oxyethylenierte C₁₂-C₂₀-Fettalkohole,
- polycyclische Alkohole, wie Sterole,
- aliphatische Alkohole mit einem hohen Molekulargewicht, wie Lanolin,
- Mischungen von oxypropylenierten/polyglycerinierten Alkoholen und Magnesiumisostearat,
- Succinester von polyoxyethylierten oder polyoxypropylenierten Fettalkoholen,
- Magnesium-, Calcium-, Lithium-, Zink-oder Aluminiumlanolat und-stearat, gegebenenfalls als Mischung mit hydriertem Lanolin, Lanölinalkohol, oder Stearinsäure oder Stearylalkohol.

Als besonders vorteilhaft haben sich Emulgatoren der Span^{®}-Reihe (ICI Americas, Inc.) herausgestellt. Dabei handelt es sich um teilweise mehrfach mit einer Fettsäure verestertes cyclisiertes Sorbit, wobei das Grundgerüst noch mit weiteren, von oberflächenaktiven Verbindungen bekannten Resten substituiert sein kann, beispielsweise mit Polyoxyethylen. Beispielhaft seien die Sorbitanester mit Laurin-, Palmitin-, Stearin- und Ölsäure erwähnt wie Span 80 (Sorbitanmonooleat) und Span 60 (Sorbitanmonostearat).

In einer bevorzugten Ausführungsform werden oxypropylenierte/ oxyethylenierte C₁₂-C₂₀-Fettalkohole als Mischungskomponente mit weiteren oberflächenaktiven Substanzen eingesetzt. Diese Fettalkohole haben in der Regel 3 bis 12 Ethylenoxid bzw. Propylenoxid-Einheiten.

Bevorzugt verwendet man als Emulgator C₁₂-C₁₈-Sorbitanfettsäureester. Diese können einzeln, in ihren Mischungen und/oder als Mischungen mit anderen obengenannten Emulgatortypen eingesetzt werden. Bevorzugt beträgt der Anteil an Sorbitanfettsäureester 20-100 Gew.-% der Gesamtmenge an eingesetzter oberflächenaktiver Substanz.

In einer bevorzugten Ausführungsform wählt man eine Mischung von oberflächenaktiven Substanzen enthaltend die oben definierte linearen Blockcopolymere und C₁₂-C₁₈-Sorbitanfettsäureester.

Besonders bevorzugt wählt man eine Mischung oberflächenaktiver Substanzen enthaltend die linearen Blockcopolymere C₁₂-C₁₈-Sorbitanfettsäureester und oxypropylenierte/oxyethylenierteC₁₂-C₂₀-Fettalkohole.

Bevorzugt werden solche Mischungen enthaltend 20 bis 95 Gew.-% insbesondere 30 bis 75 Gew.-% lineares Blockcopolymer und 5 bis 80 Gew. -% insbesondere 25 bis 70 Gew.-% C₁₂-C₁₈-Sorbitanfettsäureester bezogen auf die Gesamtmenge oberflächenaktive Substanz. Der Anteil an oxypropylenierten/oxyethyleniertenC₁₂-C₂₀-Fettalkohol beträgt bevorzugt 0 bis 20 Gew.-%.

Insbesondere werden Mischungen von oberflächenaktiven Substanzen bevorzugt enthaltend im Wesentlichen 40 bis 60 Gew.-% lineares Blockcopolymer, 30 bis 50 Gew.-% C₁₂-C₁₈-Sorbitanfettsäureester und 2 bis 10 Gew. -% oxypropylenierte/oxyethylenierte C₁₂-C₂₀-Fettalkohole, bezogen auf die Gesamtmenge oberflächenaktive Substanz.

Die optimale Menge an oberflächenaktiver Substanz wird zum einen von der oberflächenaktiven Substanz selbst, zum anderen von der Reaktionstemperatur, der gewünschten Mikrokapselgröße und den Wandmaterialien beeinflusst. Durch einfache Reihenversuche kann die optimal benötigte Menge leicht ermittelt werden. In der Regel wird die oberflächenaktive Substanz zur Herstellung der Emulsion in einer Menge von 0,01 bis 10 Gew. -%, bevorzugt 0,05 bis 5 Gew. -% und insbesondere 0,1 bis 3 Gew.-% bezogen auf die hydrophobe Phase angewendet.

Als Polymerisationsinitiatoren können sämtliche unter den Polymerisationsbedingungen in Radikale zerfallende Verbindungen eingesetzt werden, z. B. Peroxide, Hydroperoxide, Persulfate, Azoverbindungen und die sogenannten Redoxinitiatoren.

In manchen Fällen ist es vorteilhaft, Mischungen verschiedener Polymerisationsinitiatoren zu verwenden, z. B. Mischungen aus Wasserstoffperoxid und Natrium- oder Kaliumperoxodisulfat. Mischungen aus Wasserstoffperoxid und Natriumperoxodisulfat können in jedem beliebigen Verhältnis verwendet werden. Geeignete organische Peroxide sind beispielsweise Acetylacetonperoxid, Methylethylketonperoxid, tert.-Butylhydroperoxid, Cumolhydroperoxid, tert.-Amylperpivalat, tert.-Butylperpivalat, tert.-Butylperneohexanoat, tert.-Butylperisobutyrat, tert.-Butylper-2-ethylhexanoat, tert.-Butylperisononanoat, tert.-Butylpermaleat, tert.-Butylperbenzoat, tert.-Butylper-3,5,5-trimethylhexanoat und tert.-Amylperneodekanoat. Weitere geeignete Polymerisationsinitiatoren sind Azostarter, z. B. 2,2'-Azobis-(2-amidinopropan)dihydrochlorid, 2,2'-Azobis-(N,N-dimethylen)isobutyramidin-dihydrochlorid, 2-(Carbamoylazo)isobutyronitril und 4,4'-Azobis-(4-cyanovaleriansäure).

Bevorzugt ist der Einsatz von Azostartern und Peroxiden als Polymerisationsinitiatoren. Die genannten Polymerisationsinitiatoren werden in üblichen Mengen eingesetzt, z. B. in Mengen von 0,1 bis 5, vorzugsweise 0,1 bis 2,5 Mol-%, bezogen auf die zu polymerisierenden Monomere.

Die Dispergierung des Kernmaterials erfolgt je nach der Größe der herzustellenden Kapseln in bekannter Weise. Für die Herstellung großer Kapseln reicht die Dispergierung unter Verwendung von wirksamen Rührern, insbesondere von Anker- und MIG (Kreuzbalken)-Rührern aus. Kleine Kapseln, insbesondere wenn die Größe unterhalb von 50 µm liegen soll, erfordern Homogenisier- oder Dipergiermaschinen.

Die Kapselgröße kann über die Tourenzahl des Dispergiergerätes/ Homogenisiergerätes und/oder mit Hilfe der Konzentration der oberflächenaktiven Substanz bzw. über dessen Molekulargewicht, d. h. über die Viskosität der kontinuierlichen Phase innerhalb gewisser Grenzen gesteuert werden. Dabei nimmt mit Erhöhung der Tourenzahl bis zu einer Grenztourenzahl die Größe der dispergierten Teilchen ab.

Dabei ist es wichtig, dass die Dispergiergeräte zu Beginn der Kapselbildung angewendet werden. Bei kontinuierlich arbeitenden Geräten mit Zwangsdurchlauf ist es vorteilhaft, die Emulsion mehrmals durch das Scherfeld zu schicken.

In der Regel führt man die Polymerisation bei 20 bis 100°C, vorzugsweise bei 40 bis 95°C durch.
Zweckmäßigerweise wird die Polymerisation bei Normaldruck vorgenommen, jedoch kann man auch bei vermindertem oder leicht erhöhtem Druck z. B. bei einer Polymerisationstemperatur oberhalb 100°C, arbeiten, also etwa im Bereich von 0,5 bis 5 bar.

Die Reaktionszeiten der Polymerisation betragen normalerweise 1 bis 10 Stunden, meistens 2 bis 5 Stunden.

Nach dem erfindungsgemäßen Verfahren können Mikrokapseldispersionen mit einem Gehalt von 5 bis 40 Gew.-% an Mikrokapseln hergestellt werden. Die Mikrokapseln sind Einzelkapseln. Durch geeignete Bedingungen bei der Dispergierung können Kapseln mit einer mittleren Teilchengröße im Bereich von 0,5 bis zu 100 µm hergestellt werden. Bevorzugt werden Kapseln mit einer mittleren Teilchengröße von 0,5 bis 50 µm, insbesondere bis 20 µm.

Das erfindungsgemäße Verfahren ermöglicht die Herstellung von Mikrokapseln mit einem hydrophilen Kapselkern und einer Kapselwand aus einem Polymer auf Basis von (Meth)acrylsäureestern. Die erfindungsgemäßen Kapseln lassen sich je nach Kernmaterial in den verschiedensten Gebieten einsetzten. Auf diesem Weg ist es möglich hydrophile Flüssigkeiten oder Mischungen organischer Säuren oder deren Salze, anorganische Säuren, anorganische Basen, Salze anorganischer Säuren, wasserlösliche Farbstoffe, Geschmacksstoffe, pharmazeutische Wirkstoffe, Dünger, Pflanzenschutzwirkstoffe oder kosmetische Wirkstoffe in eine feste Formulierung bzw. öldisperigerbare Formulierung überführen, die diese nach Bedarf freisetzt.

So sind Mikrokapseln mit einem Wasserkern geeignet als Porenbildner für Beton. Eine weitere Anwendung in Baumaterialien ist der Einsatz von verkapselten wasserlöslichen Katalysatoren in Bindebaustoffen.

Mikrokapseln mit verkapselten anorganischen oder organischen Säuren können vorteilhaft als Bohrhilfsmittel für beispielsweise Geothermiebohrungen eingesetzt werden, da sie eine Freisetzung erst am Bohrort ermöglichen. So ermöglichen sie die Erhöhung der Permeabilität von unterirdischen, carbonatischen erdöl- und/oder erdgasführenden und/oder hydrothermalen Gesteinsformationen und zum Lösen von carbonatischen und/oder carbonathaltigen Verunreinigungen bei der Förderung von Erdöl und/oder Erdgas oder der Energiegewinnung durch hydrothermale Geothermie, indem man eine Formulierung enthaltend erfindungsgemäße Mikrokapseln mit verkapselten anorganischen oder organischen Säuren durch mindestens eine Bohrung in die Gesteinsformation einpresst. Ferner sind verkapselte Säuren, die ja eine verzögerte oder gezielte Freisetzung der Säure ermöglichen, auch als Katalysatoren zur Herstellung von Pressspanplatten geeignet.

Ferner ermöglicht die erfindungsgemäße Mikrokapseldispersion mit wasserlöslichen Bleichmittel oder Enzymen als Kernmaterial den Einsatz in Wasch- und Reinigungsmitteln insbesondere in Flüssigformulierungen. Somit betrifft die vorliegende Erfindung auch die Verwendung der Mikrokapseldispersion in Waschmitteln für Textilien und Reinigungsmitteln für nicht textile Oberflächen.

Ferner können Wirkstoffe, die kontrolliert freigesetzt werden sollen seien es medizinische Wirkstoffe, kosmetische Wirkstoffe oder auch Pflanzenschutzwirkstoffe so zubereitet werden, da bedingt durch die Dichtigkeit der Kapselwand eine Freisetzung über einen längeren Zeitraum erfolgt.

### Beispiele

### Beispiel 1

**Ölphase:**

| | |
|---|---|
| 495,42 g | Miglyol^{®} 812(Decanoyl/Octanoylglycerid Fettsäureester; Fa.Hüls) |
| 4,55 g | Arlacel® P 135 (PEG-30 Dipolyhydroxystearat, Atlas Chemie) |
| 1,19 g | Cremophor A 6 [75 Gew. -% Ceteareth-6 (ethoxilierter Cetylalkohol)] |
| 1,19 g | Span® 80 (Sorbitanmonooleat) |
| 4,55 g | Span 85 (Sorbitantrioleat) |
| 12,00 g | Methylmethacrylat |
| 8,00 g | 1,4-Butandioldiacrylat |

**Zulauf 1**

| | |
|---|---|
| 160,00 g | Wasser (Kernmaterial) |
| 20,00 g | N-Maltoyl-N-methyl-methacrylamid |

**Zulauf 2**

| | |
|---|---|
| 1,33 g | einer 75 gew.-%igen wässrigen Lösung von tert.-Butylperpivalat |

Die Ölphase wurde vorgelegt, Zulauf 1 wurde zugegeben und es wurde für 30 Minuten mit einem schnelllaufenden Dissolverrührer (Scheiben-Durchmesser 5 cm) bei 5000 Upm dispergiert. Anschließend wurde Zulauf 2 zugegeben. Die Emulsion wurde unter Rühren mit einem Ankerrüher in 60 Minuten auf 60 °C aufgeheizt. Über 120 Minuten wurde die Temperatur auf 70 °C erhöht und in weiteren 30 Minuten auf 85 °C erwärmt. Anschließend wurde 120 Minuten bei dieser Temperatur gerührt. Dann wurde auf Raumtemperatur abgekühlt.
Es wurde eine ölbasierte Mikrokapseldispersion mit einer mittleren Teilchengröße D [4,3] von < 1 µm erhalten. Die Wandstärke der Mikrokapseln betrug 20 Gew.-% und der Feststoffgehalt der Mikrokapseldispersion betrug 30 Gew.-%.

### Beispiel 2

**.Ölphase:**

| | |
|---|---|
| 495,42 g | Diisopropylnaphthalin |
| 4,55 g | Arlacel P 135 |
| 1,19 g | Cremophor A 6 |
| 1,19 g | Span 80 |
| 4,55 g | Span 85 |
| 12,00 g | Methylmethacrylat (MMA) |
| 8,00 g | 1,4-Butandioldiacrylat (BDDA) |

**Zulauf 1:**

| | |
|---|---|
| 100,00 g | Wasser (Kernmaterial) |
| 60,00 g | Maleinsäure |
| 20,00 g | N-Allylgluconamid |

**Zulauf 2:**

| | |
|---|---|
| 1,33 g | einer 75 gew.-%igen wässrigen Lösung von tert.-Butylperpivalat |

Die Ölphase wurde vorgelegt, Zulauf 1 wurde zugegeben und es wurde für 30 Minuten mit einem schnelllaufenden Dissolverrührer (Scheiben-Durchmesser 5 cm) bei 5000 Upm dispergiert. Zulauf 2 wurde zugegeben. Die Emulsion wurde unter Rühren mit einem Ankerrüher in 60 Minuten auf 60 °C aufgeheizt. Über 120 Minuten wurde die Temperatur auf 70 °C erhöht und in weiteren 30 Minuten auf 85 °C erwärmt. Anschließend für 120 Minuten, bei dieser Temperatur gerührt. Dann wurde auf Raumtemperatur abgekühlt.
Es wurde eine ölbasierte Mikrokapseldispersion mit einer mittleren Teilchengröße D [4,3] von < 1 µm erhalten. Die Wandstärke der Mikrokapseln betrug 20 Gew.-%. Der Feststoffgehalt der Mikrokapseldispersion betrug 30 Gew.-%.

### Beispiel 3

**Ölphase:**

| | |
|---|---|
| 608,77 g | Diisopropylnaphthalin |
| 10,00 g | Atlox^{®} 4912 |
| 12,50 g | Methylmethacrylat (MMA) |

**Zulauf 1:**

| | |
|---|---|
| 225,00 g | Wasser |
| 7,73 g | einer 97%igen wässrigen Lösung von 2-Hydroxyethylmethacrylat (HEMA) |
| 1,00 g | Natriumperoxodisulfat |
| 5,00 g | eines C₁₆/₁₈ Fettalkoholpolyglycolethers (Lutensol AT 25) |

Die Ölphase wurde bei 40 °C vorgelegt, Zulauf 1 wurde zugegeben und es wurde für 30 Minuten mit einem schnelllaufenden Dissolverrührer (Scheiben-Durchmesser 5 cm) bei 3000 Upm dispergiert. Zulauf 2 wurde zugegeben. Die Emulsion wurde unter Rühren mit einem Ankerrüher in 60 Minuten auf 60 °C aufgeheizt. Über 120 Minuten wurde die Temperatur auf 70 °C erhöht und in weiteren 30 Minuten auf 85 °C erwärmt. Anschließend wurde 120 Minuten bei dieser Temperatur gerührt. Dann wurde auf Raumtemperatur abgekühlt.
Es wurde eine ölbasierte Mikrokapseldispersion mit einer mittleren Teilchengröße D [4,3] von < 1 µm erhalten. Die Wandstärke der Mikrokapseln betrug 7,75 Gew.-% und der Feststoffgehalt der Mikrokapseldispersion betrug 30 Gew.-%.

### Beispiel 4

**Ölphase:**

| | |
|---|---|
| 608,69 g | Diisopropylnaphthalin |
| 5,00 g | Atlox 4912 |
| 15,00 g | Methylmethacrylat (MMA) |

**Zulauf 1:**

| | |
|---|---|
| 225,00 g | Wasser |
| 10,31 g | einer 97 gew.-%igen wässrigen Lösung von 2-Hydroxyethylmethacrylat (HEMA) |
| 1,00 g | Natriumperoxodisulfat |

Die Ölphase wurde vorgelegt, Zulauf 1 wurde zugegeben und es wurde für 20 Minuten mit einem schnelllaufenden Dissolverrührer (Scheiben-Durchmesser 5 cm) bei 3000 Upm dispergiert. Die Emulsion wurde unter Rühren mit einem Ankerrüher in 60 Minuten auf 60 °C aufgeheizt. Über 120 Minuten wurde die Temperatur auf 70 °C erhöht und in weiteren 30 Minuten auf 85 °C erwärmt. Anschließend wurde 120 Minuten bei dieser Temperatur gerührt. Dann wurde auf Raumtemperatur abgekühlt.
Es wurde eine ölbasierte Mikrokapseldispersion mit einer mittleren Teilchengröße D [4,3] von < 1 µm erhalten. Die Wandstärke der Mikrokapseln betrug 10 Gew.-% bezogen auf Wand und Kern. Der Feststoffgehalt der Mikrokapseldispersion betrug 30 Gew.-%.

### Beispiel 5

**Ölphase:**

| | |
|---|---|
| 453,68 g | Diisopropylnaphthalin |
| 1,50 g | Atlox 4912 |
| 18,00 g | Methylmethacrylat (MMA) |

**Zulauf 1:**

| | |
|---|---|
| 270,00 g | Wasser |
| 12,37 g | einer 97 gew.-%igen wässrigen Lösung von 2-Hydroxyethylmethacrylat (HEMA) |
| 1,20 g | Natriumperoxodisulfat |

Die Ölphase wurde vorgelegt, Zulauf 1 wurde zugegeben und es wurde für 10 Minuten mit einem schnelllaufenden Dissolverrührer (Scheiben-Durchmesser 5 cm) bei 2000 Upm dispergiert. Die Emulsion wurde unter Rühren mit einem Ankerrüher in 60 Minuten auf 60 °C aufgeheizt. Über 120 Minuten wurde die Temperatur auf 70 °C erhöht und in weiteren 30 Minuten auf 85 °C erwärmt. Anschließend wurde 120 Minuten bei dieser Temperatur gerührt. Dann wurde auf Raumtemperatur abgekühlt. Die Wandstärke der Mikrokapseln betrug 10 Gew.-% der Mikrokapsel. Der Feststoffgehalt der Mikrokapseldispersion betrug 40 Gew.-%.

### Beispiel 6

**Ölphase:**

| | |
|---|---|
| 800,00 g | Diisopropylnaphthalin |
| 8,00 g | Atlox 4912 |

**Zulauf 1:**

| | |
|---|---|
| 205,70 g | einer 35 %igen Natriumsilikatlösung in Wasser |
| 154,30 g | Wasser |

**Zulauf 2:**

| | |
|---|---|
| 34,00 g | Methylmethacrylat (MMA) |
| 4,00 g | 1,4-Butandioldiacrylat |
| 2,00 g | 2-Hydroxyethylmethacrylat |

**Zulauf 3:**

| | |
|---|---|
| 0,15 g | Wako V 50 [2,2'-Azobis(2-amidinoproprane) dihydrochlorid] |

**Zulauf 4:**

| | |
|---|---|
| 0,15 g | Wako V 65 [2,2'-Azobis(2,4-dimethylvaleronitril)] |

Die Ölphase wurde vorgelegt, Zulauf 3 wurde in Zulauf 1 gelöst und Zuläufe 1 und 2 wurden zur Ölphase zugegeben. Es wurde für 20 Minuten mit einem schnelllaufenden Dissolverrührer (Scheiben-Durchmesser 5 cm) bei 2000 Upm dispergiert und anschließend wurde Zulauf 4 zugegeben. Die Emulsion wurde unter Rühren mit einem Ankerrüher in 60 Minuten auf 67 °C aufgeheizt und in weiteren 60 Minuten auf 75 °C. Anschließend wurde 180 Minuten bei dieser Temperatur gerührt. Dann wurde auf Raumtemperatur abgekühlt. Die Wandstärke der Mikrokapseln betrug 10 Gew.-% der Mikrokapsel. Der Feststoffgehalt der Mikrokapseldispersion betrug 34 Gew.-%.

### Beispiel 7

Analog Beispiel 2 wurde anstelle der Mischung aus Maleinsäure und Wasser stattdessen eine Mischung aus 70, 59 g Phosphorsäure und 89,41 g Wasser verkapselt.
Die Wandstärke der Mikrokapseln betrug 20 Gew.-% der Mikrokapsel. Der Feststoffgehalt der Mikrokapseldispersion betrug 30 Gew.-%.

### Beispiel 8

Analog Beispiel 2 wurde anstelle der Mischung aus Maleinsäure und Wasser stattdessen 60,00 g Catechol mit 100,00 g Wasser verkapselt.
Die Wandstärke der Mikrokapseln betrug 20 Gew.-% der Mikrokapsel. Der Feststoffgehalt der Mikrokapseldispersion betrug 30 Gew.-%.

### Beispiel 9

Analog Beispiel 3 wurde eine Mikrokapseldispersion hergestellt wobei als Ölphase eine Mischung aus

| | |
|---|---|
| 597,10 g | Diisopropylnaphthalin |
| 5,00 g | Atlox^{®} 4912 |
| 12,50 g | Methylmethacrylat (MMA) eingesetzt wurde. |

Die Wandstärke der Mikrokapseln betrug 7,75 Gew.-% der Mikrokapsel. Der Feststoffgehalt der Mikrokapseldispersion betrug 30 Gew.-%.

### Beispiel 10

Analog Beispiel 4 wurde eine Mikrokapseldispersion hergestellt wobei als Zulauf 1 eine Mischung aus

| | |
|---|---|
| 225,00 g | Wasser |
| 10,00 g | 2-Hydroxyethylacrylat |
| 1,00 g | Natriumperoxodisulfat |

eingesetzt wurde.

Die Wandstärke der Mikrokapseln betrug 10 Gew.-% der Mikrokapsel. Der Feststoffgehalt der Mikrokapseldispersion betrug 29,6 Gew.-%.

### Beispiel 11

Analog Beispiel 4 wurde eine Mikrokapseldispersion hergestellt, wobei die Ölphase folgende Zusammensetzung hatte.

**Ölphase:**

| | |
|---|---|
| 588,27 g | Diisopropylnaphthalin |
| 1,25g | Atlox 4912 |
| 10,00 g | Methylmethacrylat (MMA) |
| 5,00 g | 1,4-Butandioldiacrylat |

Die Wandstärke der Mikrokapseln betrug 10 Gew.-% der Mikrokapsel. Der Feststoffgehalt der Mikrokapseldispersion betrug 30 Gew.-%.

### Beispiel 12

**Ölphase:**

| | |
|---|---|
| 495,42 g | Diisopropylnaphthalin |
| 4,55 g | Arlacel P 135 |
| 1,19 g | Cremophor A 6 |
| 1,19 g | Span 80 |
| 4,55 g | Span 85 |
| 12,00 g | Methylmethacrylat (MMA) |
| 8,00 g | 1,4-Butandioldiacrylat (BDDA) |

**Zulauf 1:**

| | |
|---|---|
| 89,41 g | Wasser (Kernmaterial) |
| 70,59 g | Phosphorsäure |
| 20,00 g | 1-Methacrylamido-2-D-gluconoylaminoethan |

**Zulauf 2:**

| | |
|---|---|
| 1,33 g | einer 75 gew.-%igen wässrigen Lösung von tert.-Butylperpivalat |

Die Ölphase wurde vorgelegt, Zulauf 1 wurde zugegeben und es wurde für 30 Minuten mit einem schnelllaufenden Dissolverrührer (Scheiben-Durchmesser 5 cm) bei 5000 Upm dispergiert. Zulauf 2 wurde zugegeben. Die Emulsion wurde unter Rühren mit einem Ankerrüher in 60 Minuten auf 60 °C aufgeheizt. Über 120 Minuten wurde die Temperatur auf 70 °C erhöht und in weiteren 30 Minuten auf 85 °C erwärmt. Anschließend für 120 Minuten, bei dieser Temperatur gerührt. Dann wurde auf Raumtemperatur abgekühlt.
Es wurde eine ölbasierte Mikrokapseldispersion mit einer mittleren Teilchengröße D [4,3] von < 1 µm erhalten. Die Wandstärke der Mikrokapseln betrug 20 Gew.-%. Der Feststoffgehalt der Mikrokapseldispersion betrug 30 Gew.-%.

### Beispiel 13

Analog Beispiel 4, aber mit 1,00 g Wako V50 anstatt Natriumperoxodisulfat und mit der in Beispiel 11 beschriebenen Ölphase wurde eine Mikrokapseldispersion hergestellt.

Die Wandstärke der Mikrokapseln betrug 10 Gew.-% der Mikrokapsel. Der Feststoffgehalt der Mikrokapseldispersion betrug 30 Gew.-%.

### Beispiel 14

**Ölphase:**

| | |
|---|---|
| 588,27 g | Diisopropylnaphthalin |
| 1,25 g | Atlox 4912 |
| 7,50 g | Methylmethacrylat (MMA) |
| 10,00 g | tert.-Butylacrylat |

**Zulauf 1:**

| | |
|---|---|
| 225,00 g | Wasser |
| 7,73 g | einer 97 gew.-%igen wässrigen Lösung von 2-Hydroxyethylmethacrylat (HEMA) |
| 1,00 g | Wako V 50 |

Die Ölphase wurde vorgelegt, Zulauf 1 wurde zugegeben und es wurde für 10 Minuten mit einem schnelllaufenden Dissolverrührer (Scheiben-Durchmesser 5 cm) bei 2000 Upm dispergiert. Die Emulsion wurde unter Rühren mit einem Ankerrüher in 60 Minuten auf 60 °C aufgeheizt. Über 120 Minuten wurde die Temperatur auf 70 °C erhöht und in weiteren 30 Minuten auf 85 °C erwärmt. Anschließend wurde 120 Minuten bei dieser Temperatur gerührt. Dann wurde auf Raumtemperatur abgekühlt.
Es wurde eine ölbasierte Mikrokapseldispersion mit einer mittleren Teilchengröße D [4,3] von < 1 µm erhalten. Die Wandstärke der Mikrokapseln betrug 10 Gew.-% bezogen auf Wand und Kern. Der Feststoffgehalt der Mikrokapseldispersion betrug 30 Gew.-%.

### Beispiel 15

Analog Beispiel 14 wurde anstelle von 10,00 g tert-Butylacrylat 10,00 g Glycidylmethacrylat eingesetzt.
Die Wandstärke der Mikrokapseln betrug 10 Gew.-% und der Feststoffgehalt der Mikrokapseldispersion betrug 30 Gew.-%.

## Patentansprüche

1. Mikrokapseldispersion enthaltend Mikrokapseln umfassend einen hydrophilen Kapselkern und ein Kapselwandpolymer, das erhältlich ist durch Polymerisation einer Monomerzusammensetzung umfassend
| | |
|---|---|
| 25 bis 95 Gew.-% | eines oder mehrerer C₁-C₂₄-Alkyl- und/oder Glycidylester der Acryl- und/oder Methacrylsäure |
| 5 bis 75 Gew.-% | eines oder mehrerer hydrophiler Monomere ausgewählt unter Acrylsäure- und/oder Methacrylsäureestern, die Hydroxy und/oder Carboxygruppen tragen, und Allylgluconamid |
| 0 bis 40 Gew.-% | einer oder mehrerer Verbindungen mit zwei oder mehr ethylenisch ungesättigten Resten, |
wobei die Mikrokapseln in einem hydrophoben Verdünnungsmittel dispergiert sind.

2. Mikrokapseldispersion nach Anspruch 1, **dadurch gekennzeichnet, dass** der hydrophile Kapselkern der Mikrokapseln ausgewählt wird unter Wasser sowie wässrigen Lösungen von organischen Säuren sowie deren Salzen, anorganischen Säuren und anorganischen Salzen und Natriumsilikat.

3. Mikrokapseldispersion nach Anspruch 1 oder 2, **dadurch gekennzeichnet, dass** die Monomerzusammensetzung Methylmethacrylat enthält.

4. Mikrokapseldispersion nach einem der Ansprüche 1 bis 3, **dadurch gekennzeichnet, dass** das hydrophile Monomer ausgewählt wird unter Hydroxyalkylacrylaten, Hydroxyalkylmethacrylaten, Acrylamidoalkyl-polyhydroxysäureamiden, Methacrylamidoalkyl-polyhydroxysäureamiden, N-Acryl-glykosylaminen und N-Methacryl-glykosylaminen.

5. Mikrokapseldispersion nach einem der Ansprüche 1 bis 4, erhältlich durch Herstellen einer Wasser-in-Öl-Emulsion enthaltend hydrophobes Verdünnungsmittel als kontinuierliche Phase sowie das hydrophile Kapselkernmaterial und die Monomerzusammensetzung und anschließender radikalischer Polymerisation der Monomere zur Bildung des Kapselwandpolymers.

6. Mikrokapseldispersion nach einem der Ansprüche 1 bis 5, **dadurch gekennzeichnet, dass** das hydrophobe Verdünnungsmittel eine Löslichkeit in Wasser < 0,5g/L bei 20°C und Normaldruck aufweist.

7. Verfahren zur Herstellung einer Mikrokapseldispersion nach einem der Ansprüche 1 bis 6, **dadurch gekennzeichnet, dass** man eine Wasser-in-Öl-Emulsion enthaltend ein hydrophobes Verdünnungsmittel als kontinuierliche Phase sowie das hydrophile Kapselkernmaterial und die Monomerzusammensetzung herstellt und anschließende die Monomere radikalisch polymerisiert, wobei die Monomerzusammensetzung
| | |
|---|---|
| 25 bis 95 Gew.-% | eines oder mehrerer C₁-C₂₄-Alkylester der Acryl- und/oder Methacrylsäure, |
| 5 bis 75 Gew.-% | eines oder mehrerer Monomere ausgewählt unter Acrylsäure- und/oder Methacrylsäureestern, die Hydroxy und/oder Carboxygruppen tragen, und Allylgluconamid |
| 0 bis 40 Gew.-% | einer oder mehrerer Verbindungen mit zwei oder mehr ethylenisch ungesättigten Resten umfasst. |

8. Verfahren nach Anspruch 7, **dadurch gekennzeichnet, dass** man die Wasser-in-Öl-Emulsion mit einer oberflächenaktiven Substanz stabilisiert, die ein lineares Blockcopolymer ist mit einer hydrophoben Struktureinheit von einer Länge von mehr als 50Å und welches durch die allgemeinen Formel
C_{w}-(-B-A-B_{y}-)-x D_{z}
definiert wird, in der
w 0 oder 1 ist,
x 1 oder mehr ist,
y 0 oder 1 ist, und
z 0 der 1 ist
A eine hydrophile Struktureinheit ist, die eine Molmasse von 200 bis 50 000 aufweist mit einer Löslichkeit in Wasser bei 25 °C > 1 Gew.-%, und so ausgewählt ist, dass sie kovaltent an B gebunden wird, und
B eine hydrophobe Struktureinheit ist, die eine Molmasse von 300 bis 60 000 und eine Löslichkeit in Wasser bei 25°C von <1 % hat und kovalent an A gebunden werden kann, und
C und D Endgruppe sind, die unabhängig voneinander A oder B sein können.

9. Verfahren nach Anspruch 8, **dadurch gekennzeichnet, dass** man die Wasser-in-Öl-Emulsion mit einem 12-Hydroxystearinsäure-Blockcopolymer als linearem Blockcopolymer stabilisiert.

10. Verfahren nach Anspruch 8, **dadurch gekennzeichnet, dass** man die Wasser-in-Öl-Emulsion mit C₁₂-C₁₈-Sorbitanfettsäureester als oberflächenaktive Substanz stabilisiert.

11. Verwendung der Mikrokapseldispersion gemäß den Ansprüchen 1 bis 6 enthaltend Wasser oder anorganische Säuren als Hilfsmittel zur Modifizierung von Bindebaustoffen.

12. Verwendung der Mikrokapseldispersion gemäß den Ansprüchen 1 bis 6 mit einem kosmetischen Wirkstoff als Kernmaterial als Bestandteil in kosmetischen Zubereitungen.

13. Verwendung der Mikrokapseldispersion gemäß den Ansprüchen 1 bis 6 mit Pflanzenschutzwirkstoffen als Kernmaterial als Bestandteil in agrochemischen Formulierungen.

## Claims

1. A microcapsule dispersion comprising microcapsules comprising a hydrophilic capsule core and a capsule wall polymer which is obtainable by polymerization of a monomer composition comprising
| | |
|---|---|
| 25 to 95% by weight | of one or more C₁-C₂₄-alkyl and/or glycidyl esters of acrylic acid and/or methacrylic acid |
| 5 to 75% by weight of | one or more hydrophilic monomers selected from acrylic acid esters and/or methacrylic acid esters which carry hydroxy and/or carboxy groups, and allylgluconamide |
| 0 to 40% by weight of | one or more compounds having two or more ethylenically unsaturated radicals, |
where the microcapsules are dispersed in a hydrophobic diluent.

2. The microcapsule dispersion according to claim 1, wherein the hydrophilic capsule core of the microcapsules is selected from water, and aqueous solutions of organic acids, and salts thereof, inorganic acids and inorganic salts and of sodium silicate.

3. The microcapsule dispersion according to claim 1 or 2, wherein the monomer composition comprises methyl methacrylate.

4. The microcapsule dispersion according to any one of claims 1 to 3, wherein the hydrophilic monomer is selected from hydroxyalkyl acrylates, hydroxyalkyl methacrylates, acrylamidoalkyl-polyhydroxy acid amides, methacrylamidoalkyl-polyhydroxy acid amides, N-acryl-glycosylamines and N-methacrylglycosylamines.

5. The microcapsule dispersion according to any one of claims 1 to 4, obtainable by preparing a water-in-oil emulsion comprising hydrophobic diluent as continuous phase, and the hydrophilic capsule core material and the monomer composition and subsequent free-radical polymerization of the monomers to form the capsule wall polymer.

6. The microcapsule dispersion according to any one of claims 1 to 5, wherein the hydrophobic diluent has a solubility in water < 0.5g/l at 20°C and atmospheric pressure.

7. A method for producing a microcapsule dispersion according to any one of claims 1 to 6, wherein a water-in-oil emulsion comprising a hydrophobic diluent as continuous phase, and the hydrophilic capsule core material and the monomer composition is prepared and then the monomers are free-radically polymerized, the monomer composition comprising
| | |
|---|---|
| 25 to 95% by weight | of one or more C₁-C₂₄-alkyl and/or glycidyl esters of acrylic acid and/or methacrylic acid |
| 5 to 75% by weight of | one or more hydrophilic monomers selected from acrylic acid esters and/or methacrylic acid esters which carry hydroxy and/or carboxy groups, and allylgluconamide |
| 0 to 40% by weight of | one or more compounds having two or more ethylenically unsaturated radicals. |

8. The method according to claim 7, wherein the water-in-oil emulsion is stabilized with a surface-active substance which is a linear block copolymer with a hydrophobic structural unit of a length of more than 50Å and which is defined by the general formula
C_{w}-(-B-A-B_{y}-)-x D_{z}
in which
w is 0 or 1,
x is 1 or more,
y is 0 or 1, and
z is 0 or 1
A is a hydrophilic structural unit which has a molar mass of from 200 to 50 000 with a solubility in water at 25 °C > 1% by weight, and is selected such that it is covalently bonded to B, and
B is a hydrophobic structural unit which has a molar mass of from 300 to 60 000 and a solubility in water at 25°C of <1% and can be covalently bonded to A, and
C and D are end groups which, independently of one another, can be A or B.

9. The method according to claim 8, wherein the water-in-oil emulsion is stabilized with a 12-hydroxystearic acid block copolymer as linear block copolymer.

10. The method according to claim 8, wherein the water-in-oil emulsion is stabilized with C₁₂-C₁₈-sorbitan fatty acid ester as surface-active substance.

11. The use of the microcapsule dispersion according to claims 1 to 6 comprising water or inorganic acids as auxiliary for modifying binding construction materials.

12. The use of the microcapsule dispersion according to claims 1 to 6 with a cosmetic active ingredient as core material as a constituent in cosmetic preparations.

13. The use of the microcapsule dispersion according to claims 1 to 6 with crop protection active ingredients as core materials as a constituent in agrochemical formulations.

## Revendications

1. Dispersion de microcapsules contenant des microcapsules comprenant un noyau de capsule hydrophile et un polymère de paroi de capsule, qui peut être obtenu par polymérisation d'une composition de monomères comprenant :
25 à 95 % en poids d'un ou de plusieurs esters alkyliques en C₁-C₂₄ et/ou glycidyliques de l'acide acrylique et/ou méthacrylique,
5 à 75 % en poids d'un ou de plusieurs monomères hydrophiles choisis parmi les esters de l'acide acrylique et/ou de l'acide méthacrylique qui portent des groupes hydroxy et/ou carboxy, et l'allylgluconamide,
0 à 40 % en poids d'un ou de plusieurs composés contenant deux radicaux éthyléniquement insaturés ou plus,
les microcapsules étant dispersées dans un diluant hydrophobe.

2. Dispersion de microcapsules selon la revendication 1, **caractérisée en ce que** le noyau de capsule hydrophile des microcapsules est choisi parmi l'eau et les solutions aqueuses d'acides organiques et leurs sels, d'acides inorganiques et de sels inorganiques et de silicate de sodium.

3. Dispersion de microcapsules selon la revendication 1 ou 2, **caractérisée en ce que** la composition de monomères contient du méthacrylate de méthyle.

4. Dispersion de microcapsules selon l'une quelconque des revendications 1 à 3, **caractérisée en ce que** le monomère hydrophile est choisi parmi les acrylates d'hydroxyalkyle, les méthacrylates d'hydroxyalkyle, les amides d'acrylamidoalkyl-polyhydroxyacides, les amides de méthacrylamidoalkyl-polyhydroxyacides, les N-acryl-glycosylamines et les N-méthacryl-glycosylamines.

5. Dispersion de microcapsules selon l'une quelconque des revendications 1 à 4, pouvant être obtenue par fabrication d'une émulsion eau dans huile contenant un diluant hydrophobe en tant que phase continue, ainsi que le matériau de noyau de capsule hydrophile et la composition de monomères, puis polymérisation radicalaire des monomères pour former le polymère de paroi de capsule.

6. Dispersion de microcapsules selon l'une quelconque des revendications 1 à 5, **caractérisée en ce que** le diluant hydrophobe présente une solubilité dans l'eau < 0,5 g/l à 20 °C et pression normale.

7. Procédé de fabrication d'une dispersion de microcapsules selon l'une quelconque des revendications 1 à 6, **caractérisé en ce qu'**une émulsion eau dans huile contenant un diluant hydrophobe en tant que phase continue, ainsi que le matériau de noyau de capsule hydrophile et la composition de monomères est fabriquée, puis les monomères sont polymérisés par voie radicalaire, la composition de monomères comprenant 25 à 95 % en poids d'un ou de plusieurs esters alkyliques en C₁-C₂₄ de l'acide acrylique et/ou méthacrylique,
5 à 75 % en poids d'un ou de plusieurs monomères hydrophiles choisis parmi les esters de l'acide acrylique et/ou de l'acide méthacrylique qui portent des groupes hydroxy et/ou carboxy, et l'allylgluconamide,
0 à 40 % en poids d'un ou de plusieurs composés contenant deux radicaux éthyléniquement insaturés ou plus.

8. Procédé selon la revendication 7, **caractérisé en ce que** l'émulsion eau dans huile est stabilisée avec une substance tensioactive, qui est un copolymère séquencé linéaire contenant une unité structurale hydrophobe d'une longueur de plus de 50 Å et qui est définie par la formule générale
C_{w}-(-B-A-B_{y}-)-x D_{z} dans laquelle
w représente 0 ou 1,
x représente 1 ou plus,
y représente 0 ou 1, t
z représente 0 ou 1,
A est une unité structurale hydrophile qui présente une masse molaire de 200 à 50 000 avec une solubilité dans l'eau à 25 °C > 1 % en poids, et qui est choisie de sorte qu'elle soit reliée à B par une liaison covalente, et
B est une unité structurale hydrophobe qui présente une masse molaire de 300 à 60 000 et une solubilité dans l'eau à 25 °C < 1 %, et qui peut être reliée à A par une liaison covalente, et
C et D sont des groupes terminaux qui peuvent représenter A ou B indépendamment l'un de l'autre.

9. Procédé selon la revendication 8, **caractérisé en ce que** l'émulsion eau dans huile est stabilisée avec un copolymère séquencé d'acide 12-hydroxystéarique en tant que copolymère séquencé linéaire.

10. Procédé selon la revendication 8, **caractérisé en ce que** l'émulsion eau dans huile est stabilisée avec un ester d'acide gras de sorbitane en C₁₂-C₁₈ en tant que substance tensioactive.

11. Utilisation de la dispersion de microcapsules selon les revendications 1 à 6 contenant de l'eau ou des acides inorganiques en tant qu'adjuvant pour la modification de matériaux de construction liants.

12. Utilisation de la dispersion de microcapsules selon les revendications 1 à 6 avec un agent actif cosmétique en tant que matériau de noyau en tant que constituant dans des préparations cosmétiques.

13. Utilisation de la dispersion de microcapsules selon les revendications 1 à 6 avec des agents actifs phytoprotecteurs en tant que matériau de noyau en tant que constituant dans des formulations agrochimiques.
